# EUROPÄISCHE PATENTSCHRIFT

(11) **EP 0 324 399 B1**
(45) Veröffentlichungstag und Bekanntmachung des Hinweises auf die Patenterteilung: **22.09.1993**
(21) Anmeldenummer: 89100234.7
(22) Anmeldetag: 07.01.1989
(51) Int. Cl.: C12N 9/14, A23L 1/03

(54) **Verfahren zur Herstellung eines macerierenden Aspergillus-Enzyms**
Process for the preparation of a macerating aspergillus enzyme
Procédé pour la préparation d'une enzyme de macération d'aspergillus

(30) Priorität: 15.01.1988 DE 3801005
(43) Veröffentlichungstag der Anmeldung: 19.07.1989
(73) Patentinhaber: Röhm GmbH, 64293 Darmstadt (DE)
(72) Erfinder: Löffler, Fridolin, Dr., D-6100 Bensheim (DE); Reiner, Roland, Dr., D-6100 Darmstadt (DE); Sprössler, Bruno, Dr., D-6101 Rossdorf 1 (DE)

(56) Entgegenhaltungen:
- DD-A- 249 919
- DE-A- 3 044 455
- FR-A- 2 021 772
- US-A- 2 479 751
- JOURNAL OF BIOTECHNOLOGY, Band 4, 1986, Seiten 269-282, Elsevier Science Publishers B.V. (Biomedical Division), Amsterdam, NL; H.K. SREENATH et al.: "The effect of removing cellulase(s) from a commercial pectinase on maceration and liquefaction of carrots"

## Beschreibung

Die Erfindung betrifft die Gewinnung eines macerierenden Enzympräparats aus einer Aspergillus-Kultur, bzw. die Auftrennung eines Aspergillus-Enzymgemisches in ein macerierendes und ein nichtmacerierendes Enzympräparat. Ein Enzympräparat wird als "macerierend" bezeichnet, wenn es bei der Einwirkung auf pflanzliches Material, wie zerkleinerte Früchte oder Gemüse, das aus Pectinstoffen, vornehmlich Protopectinen, aufgebaute interzelluläre Bindematerial auflöst und das Frucht- oder Gemüsefleisch in Einzelzellen zerlegt, ohne das hochveresterte, lösliche Pektin gleichzeitig abzubauen. Es entsteht eine stabile Suspension intakter Zellen, die als Macerat bezeichnet wird.

### Stand der Technik

Aus einer Arbeit von D. Sulc und D. Cirik in "Flüssiges Obst", Heft 6/1968 ist es bekannt, als macerierendes Enzym ein vorwiegend aus Polygalacturonasen und Protopectinasen bestehendes, von Pectinesterase und Pectin-Transeliminase freies Enzympräparat zu verwenden. Über dessen Gewinnung wird in der DE-C 1 805 808 nur angegeben, daß es aus üblichen Pectinase-Präparaten abgetrennt und angereichert werden muß; das Trennungsverfahren sei umständlich und aufwendig und in der Literatur nicht beschrieben.

Nach der DE-C 1 805 808 lassen sich macerierende Enzyme unmittelbar aus Kulturen von Asp. alleaceus, Asp. parasiticus, Rhicopus javanicus, Ceratocystis paradoxa, Pen. roqueforti und Pen. stoloniferum gewinnen.

Aus der DE-C 3 044 455 ist es bekannt, handelsübliche Pectinase-Präparate einer Alkalibehandlung zu unterwerfen, welche die Polygalacturonasen und Pectin-Transeliminasen inaktiviert und nur die Pectinesterasen aktiv läßt. Man erhält dadurch ein nichtmacerierendes Enzym.

H.K. Sreenath und B.J. Radola berichten im Journal of Biotechnology (Band 4, 1986, S. 269-282) über das Handelsprodukt Rohament P, das ein mazerierendes Enzym aus Asp. alleaceus enthält. Neben einer Endopolygalacturonase als Hauptaktivität enthält es Nebenaktivitäten von Cellulasen, Hemicellulasen, β-Glucanasen und Proteasen, die zur Auflosung der Zellwände der mazerierten Zellen führen können. Sreenath und Radola gelang es, die Nebenaktivitäten durch Affinitätschromatographie an Avicel-Cellulose so weit zu entfernen, daß in mazeriertem Karottengewebe kein Angriff der Zellwände mehr festzustellen war.

### Aufgabe und Lösung

Die Aufgabe der Erfindung bestand darin, ein neues, wirtschaftliches Verfahren zur Gewinnung von macerierenden Enzympräparaten aus Aspergillus-Kulturen zu finden.

Es wurde nun gefunden, daß sich ein macerierendes Enzympräparat herstellen läßt, wenn man eine wäßrige Lösung eines aus einer Aspergillus-Kultur gewonnenen, Pectinglykosidasen und Pectinesterase enthaltenden Enzymgemisches mit einem Kationenaustauscher in Berührung bringt, die flüssige Phase, in der sich die Hauptmenge der Pectinesterase befindet, von dem Kationenaustauscher abtrennt und von diesem das macerierende Enzympräparat eluiert.

Als Ausgangsmaterial eignen sich übliche Pektinasepräparate, die von Aspergillus-Arten gewonnen werden. Sie stammen vorwiegend von Aspergillus niger-Kulturen, insbesondere industriellen Hochleistungsstämmen, die auch erfindungsgemäß bevorzugt sind. Diese Pektinasen wirken nicht macerierend. Charakteristisch ist ein wesentlicher Gehalt an macerierender Polygalacturonase, nicht-macerierender Polygalacturonase, Pectinesterase und Pectin-Transeliminase. Beim Einsatz als Klärenzym bei der Fruchtsaftherstellung wird der natürliche Gehalt an macerierender Polygalacturonase nicht benötigt. Das Verfahren der Erfindung gestattet es nun, aus üblichen Aspergillus-Pektinasen eine Polygalacturonase als wertvolles macerierendes Enzym abzutrennen und das zurückbleibende Enzymgemisch als vollwertiges Klärenzym zu verwenden. Man gewinnt auf diese Weise durch ein einfaches Trennverfahren zwei technisch wertvolle Enzympräparate aus einer Aspergillus-Kultur.

### Ausführung der Erfindung

Eine Anzahl von Aspergillus-Arten ist als Pectinasebildner bekannt; z.B. Asp. niger, phoenices, usamii, awamori, japonicus, sojae. In der Technik werden Asp. niger-Stämme bevorzugt. Sie werden in Submers- oder Oberflächenkulturen auf Weizenkleie und Rübenschnitzeln als Nährböden gezüchtet. Das Kulturfiltrat einer Submerskultur oder der Extrakt einer Oberflächenkultur wird üblicherweise durch Ultrafiltration konzentriert und von Salzen teilweise befreit. Das Retentat, das beispielsweise eine Leitfähigkeit bis zu 20 mS (Milli-Siemens) hat, sollte gegebenenfalls durch Diafiltration auf eine Leitfähigkeit von höchstens 3,5 mS gebracht werden.

Man erhält auf diese Weise ein herkömmliches Pektinasepräparat mit einem wesentlichen Gehalt an Polygalacturonasen und Pectinesterase sowie in der Regel an Pectin-Transeliminase. Unter einem wesentlichen Gehalt an macerierender Polygalacturonase wird eine Aktivität verstanden, die bei der Anwendung auf Frucht- und Gemüsefleisch deutlich wirksam ist. Viele Schimmelpilzenzyme enthalten diese Macerase als unbedeutende Nebenaktivität, die sich beim technischen Einsatz neben den jeweiligen Hauptaktivitäten nicht deutlich bemerkbar macht. Ein typisches Pektinasepräparat, das erfindungsgemäß eingesetzt wird, enthält
Polygalacturonasen mit mindestens 10 000
Polygalacturonase (PG)-Einheiten gemäß Technischer Information der Röhm GmbH Nr. FR-5-01-D vom 12.02.1987
Pectintranseliminase mit mindestens 25 00 PTE-Einheiten gemäß Technischer Information der Röhm GmbH
Nr. FR-5-03-E vom 11.02.1987
Pectinesterase mit mindestens 100 PE-Einheiten gemäß Technischer Information der Röhm GmbH
Nr. FR-5-02-D vom 12.02.1987
Im Macerierungstest nach Sreenatz, H.K. Kogel, F. u. Radola, B.J. (1986) J. Ferment. Technol. Band 64, S. 37 - 44 erweist sich das Retentat als nicht macerierend. Im Falle eines hochwirksamen Macerationsenzyms ist das Macerat nach 24 Stunden noch trubstabil, während es sich bei Einsatz eines ungeeigneten Macerationsenzyms in einen klaren Überstand und ein Sediment auftrennt.

Das auf pH 3,5 bis 5,5, vorzugsweise etwa pH 4, gepufferte Retentat wird erfindungsgemäß mit einem Kationenaustauscher in Berührung gebracht. Herkömmliche Kationenaustauscher für die Wasserenthärtung sind zwar brauchbar, aber wegen geringer Bindungskapazität wenig geeignet. Bevorzugt sind makroporöse, hydrophile Kationenaustauscher mit einer Bindungkapazität von wenigstens 60 mg Hämoglobin oder Albumin je Milliliter Gel. Der durchschnittliche Porendurchmesser liegt vorzugsweise zwischen 0,1 - 3µm, die durchschnittliche Teilchengröße beträgt vorzugsweise 30 bis 400 Mikrometer, das Porenvolumen 40 bis 80 % des Partikelvolumens. Hydrophile Kationenaustauscher zeichnen sich durch geringe unspezifische Adsorption aus. Sie sind in Wasser wenig quellbar, vorzugsweise auf nicht mehr als das 3-fache Trockenvolumen. Eine hohe Druckstabilität ist vorteilhaft.

Die anionischen Ladungen des Kationenaustauschers können z.B. aus Carboxyl- oder Sulfonatgruppen bestehen. Ein vorteilhafter Kationenaustauscher wird erhalten, wenn man ein perlförmiges, stark vernetztes, makroporöses Mischpolymerisat des Acrylamids mit einem Gehalt an kovalent reaktiven Gruppen, wie Oxirangruppen, mit 2-Mercaptoäthansulfonsäure bzw. deren Natriumsalz umsetzt.

An diesem Kationenaustauscher wird ein erheblicher Teil, in der Regel 7 bis 25 %, vorzugsweise 10 bis 15 % der vorhandenen Polygalacturonase-Aktivität gebunden, während alle übrigen Enzymbestandteile im Durchlauf nahezu vollständig wiedergefunden werden. Neben der Pectinesterase sind das die nichtmacerierenden Polygalacturonasen und die Pectintranseliminase sowie der nicht gebundene Teil der macerierenden Polygalacturonase. Mit Ausnahme der letzteren zeichnen sich die im Durchlauf verbleibenden Enzyme durch einen isoelektrischen Punkt von 3,3 bis 3,7 aus. Der Durchlauf ist als Pectinase-Präparat für die Fruchsaftklärung unmittelbar geeignet.

Nach der Abtrennung von dem Retentat wird der Kationenaustauscher mit Pufferlösung (pH 4) gewaschen und zur Gewinnung der Polygalacturonase mit einer gepufferten Salzlösung eluiert. Man verwendet z.B. eine 1 m NaCl-, 0,01 m Na-Acetat-Lösung vom pH 4. Das Eluat wird wie üblich stabilisiert und kann unmittelbar als macerierendes Enzym eingesetzt werden. Die Polygalacturonase-Aktivität liegt in typischen Fällen zwischen 5 000 und 15 000 Polygalacturonase-Einheiten. Die Pectinesterase-Aktivität soll im Eluat unter 7 PE-Einheiten liegen, was 1 bis 5 % der ursprünglich vorhandenen Menge entspricht. Nach Pufferwäsche kann der Kationenaustauscher erneut mit Retentat beschickt werden. Zweckmäßig ist der Kationenaustauscher in einer Durchlaufsäule angeordnet.

### BEISPIEL

Eine Trennsäule wurde mit 21 l eines Kationenaustauschers auf Basis eines Perlpolymerisats aus Acrylamid, Methylenbis-methacrylamid, Glycidylmethacrylat und Allylglycidyläther, das mit Natrium-2-mercaptoethylsulfonat umgesetzt war (Handelsbezeichnung "SE-EUPERGIT", Röhm GmbH), gefüllt und mit 0,01 M Na-Acetatpuffer (pH 4) äquilibriert.

In 250 min wurden 250 kg einer wäßrigen Lösung eines Pektinasengemisches aus einem Asp.-niger-Oberflächenkulturextrakt mit einer Leitfähigkeit von 3,3 mSi und einer Polygalacturonase-Aktivität von 7,93·10¹² PG-Einheiten, aufgetragen. Mit 40 kg des oben genannten Acatatpuffers wurde nachgewaschen. Nach einem Vorlauf von 5 kg, der verworfen wurde, wurde praktisch die gesamte Menge der Pektinasen, nämlich 3,5 ·10¹² PTE-Einheiten, 59,28·10⁶ PE-Einheiten und 6,81·10¹² PG-Einheiten in 285 kg Ablauf zurückgewonnen.

Anschließend wurde die Polygalacturonase mit einer Pufferlösung, die neben der angegebenen Acetatkonzentration 1 M NaCl enthielt, in 25 kg Eluat mit insgesamt 0,49·10¹² PG-Einheiten, was 6 % der eingesetzten Menge entspricht, eluiert. Das Eluat war weitgehend frei von Pektinesterase, und -Transeliminase. Nach Regenerieren der Säule mit Acatatpuffer konnte der Prozeß mit gleichem Erfolg mehrfach wiederholt werden.

## Patentansprüche

1. Verfahren zur gleichzeitigen Herstellung eines macerierenden Enzympräparats und eines Klärenzyms für die Fruchtsaftklärung aus einem aus einer Aspergillus niger-Kultur gewonnenen Enzymgemisch mit einem wesentlichen Gehalt an macerierender Polygalacturonase, nicht-macerierender Polygalacturonase, Pectinesterase und Pectin-Transeliminase
dadurch gekennzeichnet,
daß man eine wäßrige Lösung des Enzymgemisches mit einem Kationenaustauscher in Berührung bringt, die flüssige Phase, in der sich die Hauptmenge der Pectinesterase und Pectinglykosidasen befinden, als Klärenzym von dem Kationenaustauscher abtrennt und von diesem das macerierende Enzympräparat eluiert.

2. Verfahren nach Anspruch 1, dadurch gekennzeichnet, daß eine auf pH 3,5 bis 5,5 eingestellte Enzymlösung eingesetzt wird.

3. Verfahren nach Anspruch 2, dadurch gekennzeichnet, daß eine auf etwa pH 4 eingestellte Enzymlösung eingesetzt wird.

4. Verfahren nach den Ansprüchen 1 bis 3, dadurch gekennzeichnet, daß ein makroporöser, hydrophiler Kationenaustauscher mit einer Bindungskapazität von mindestens 60 mg Hämoglobin oder Albumin/ml Gel eingesetzt wird.

## Claims

1. Process for the simultaneous production of a macerating enzyme preparation and a clarifying enzyme for clarifying fruit juice from an enzyme mixture obtained from an aspergillus niger culture, this mixture having a substantial content of macerating polygalacturonase, non-macerating polygalacturonase, pectinesterase and pectin-transeliminase, characterised in that an aqueous solution of the enzyme mixture is brought into contact with a cation exchanger, the liquid phase which contains the majority of the pectinesterase and pectinglycosydases is separated from the cation exchanger as the clarifying enzyme and from this the macerating enzyme preparation is eluted.

2. Process according to claim 1, characterised in that an enzyme solution adjusted to a pH of 3.5 to 5.5 is used.

3. Process according to claim 2, characterised in that an enzyme solution adjusted to about pH 4 is used.

4. Process according to claims 1 to 3, characterised in that a macroporous, hydrophilic cation exchanger having a binding capacity of at least 60 mg of haemoglobin or albumin per ml of gel is used.

## Revendications

1. Procédé de préparation simultanée d'une préparation enzymatique pour la macération et d'une enzyme clarifiante pour la clarification de jus de fruit, à partir d'un mélange d'enzymes obtenu à partir d'une culture d'Aspergillus niger, ce mélange ayant une teneur substantielle en polygalacturonase de macération, en polygalacturonase inapte à la macération, en pectineestérase et en pectinetranséliminase, caractérisé en ce que l'on met une solution aqueuse du mélange d'enzymes en contact avec un échangeur de cations, en ce que l'on sépare, en tant qu'enzyme clarifiante, la phase liquide de l'échangeur de cations, la majeure partie de la pectine-estérase et des pectineglucosidases se trouvant dans cette phase liquide, et en ce que l'on élue la préparation enzymatique pour la macération de cet échangeur de cations.

2. Procédé selon la revendication 1, caractérisé en ce que l'on utilise une solution d'enzymes dont le pH est compris entre 3,5 et 5,5.

3. Procédé selon la revendication 2, caractérisé en ce que l'on utilise une solution d'enzymes approximativement à pH 4.

4. Procédé selon l'une des revendications 1 à 3, caractérisé en ce que l'on utilise un échangeur de cations hydrophile, macroporeux, ayant une capacité de liaison au moins égale à 60 mg d'hémoglobine ou d'albumine/ml de gel.
